# EUROPEAN PATENT SPECIFICATION

(11) **EP 2 521 728 B1**
(45) Date of publication and mention of the grant of the patent: **07.09.2016**
(21) Application number: 10765473.3
(22) Date of filing: 06.10.2010
(51) Int. Cl.: C07D 493/04

(54) **CRYSTALLINE DARUNAVIR HYDRATE AND PROCESS FOR PREPARATION THEREOF**
KRISTALLINISCHE HYDRATE VON DARUNAVIR UND VERFAHREN ZU SEINER HERSTELLUNG
HYDRATE CRISTALLIN DE DARUNAVIR ET SON PROCÉDÉ DE PRÉPARATION

(30) Priority: 05.01.2010 IN MU00332010
(43) Date of publication of application: 14.11.2012
(73) Proprietor: Cipla Limited, Mumbai - 400013 (IN)
(72) Inventor: PHULL, Manjinder Singh, Mumbai - 400 078 Maharashtra (IN); RAO, Dharmaraj Ramachandra, Mumbai 400 601 Maharashtra (IN); KANKAN, Rajendra Narayanrao, Mumbai 400 076 Maharashtra (IN)
(74) Representative: King, Lawrence
(86) International application number: PCT/GB2010/001874
(87) International publication number: WO 2011/083287

(56) References cited:
- WO-A2-03/106461
- SURLERAUX D L N G ET AL: "Discovery and Selection of TMC114, a Next Generation HIV-1 Protease Inhibitor", JOURNAL OF MEDICINAL CHEMISTRY, AMERICAN CHEMICAL SOCIETY, US, vol. 48, no. 6, 24 March 2005 (2005-03-24) , pages 1813-1822, XP008122471, ISSN: 0022-2623, DOI: 10.1021/JM049560P [retrieved on 2004-10-05]
- Arun K. Ghosh ET AL: "Design, Synthesis, Protein-Ligand X-ray Structure, and Biological Evaluation of a Series of Novel Macrocyclic Human Immunodeficiency Virus-1 Protease Inhibitors to Combat Drug Resistance", Journal of Medicinal Chemistry, vol. 52, no. 23, 10 December 2009 (2009-12-10), pages 7689-7705, XP055110676, ISSN: 0022-2623, DOI: 10.1021/jm900695w
- VAN GYSEGHEM E ET AL: "Solid state characterization of the anti-HIV drug TMC114: Interconversion of amorphous TMC114, TMC114 ethanolate and hydrate", EUROPEAN JOURNAL OF PHARMACEUTICAL SCIENCES, ELSEVIER, AMSTERDAM, NL, vol. 38, no. 5, 8 December 2009 (2009-12-08), pages 489-497, XP026764329, ISSN: 0928-0987, DOI: 10.1016/J.EJPS.2009.09.013 [retrieved on 2009-09-24]

## Description

### Field of Invention

The present invention relates to a stable polymorphic form of darunavir. More particularly, it relates to a hydrated form of darunavir and a process for preparation thereof.

### Background and Prior Art

Darunavir, chemically known as (3R,3aS,6aR)-hexahydrofuro[2,3-b]furan-3-yl(1S,2R)-3-[[(4-amino phenyl)sulfonyl](isobutyl)amino]-1-benzyl-2-hydroxypropylcarbamate (I), is used in the treatment of HIV infection by inhibiting the viral protease enzyme.

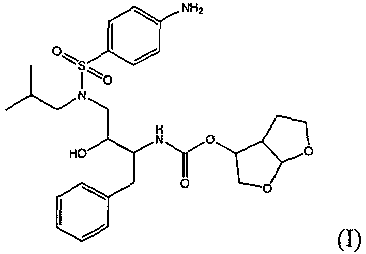

Darunavir and processes for its preparation are disclosed in EP0715618, WO9967417, EP1725566 and Bioorganic & Medicinal Chemistry Letters (2004), 14(4), 959-963.

US20050250845 discloses various pseudopolymorphs of darunavir and processes for their preparation. According to this application, "pseudopolymorph" is defined as a crystalline form of a compound in which solvent molecules are incorporated in the lattice structure. The Form B disclosed in the patent application is a pseudopolymorph wherein water is used as solvent. The thermogravimetric experiments of the Form B shows weight loss of 3.4% in the temperature range 25-78°C (water), 5.1% in the temperature range 25-110°C (ethanol and water) and further 1.1% weight loss (ethanol) in temperature range 110-200° C. Further at the drying step the Form B showed about 5.6% weight loss. The obtained dried product was hygroscopic and it adsorbed up to 6.8% water at high relative humidity.

A study of the interconversion of darunavir crystalline ethanolate, a crystalline hydrate of darunavir and amorphous darunavir is disclosed in Eur. J.Pharm. Sci. 2009, 38, 489-497.

Amorphous form of darunavir is disclosed in US20050250845 and the publication in J.Org. Chem. 2004, 69, 7822 - 7829.

### Object of the Invention

The object of the present invention is to provide stable polymorph of darunavir, free from other pseudopolymorphs or solvates, and process for its preparation.

### Summary of the Invention

The present invention relates to a polymorphic form of darunavir. More particularly the invention relates to hydrated form of darunavir.

The darunavir hydrate of the present invention is a true hydrate. The "true hydrate" hereinafter referred as 'Form C' is defined as a hydrate form which is crystalline, stable, non-hygroscopic in nature and does not contain any solvent molecule.

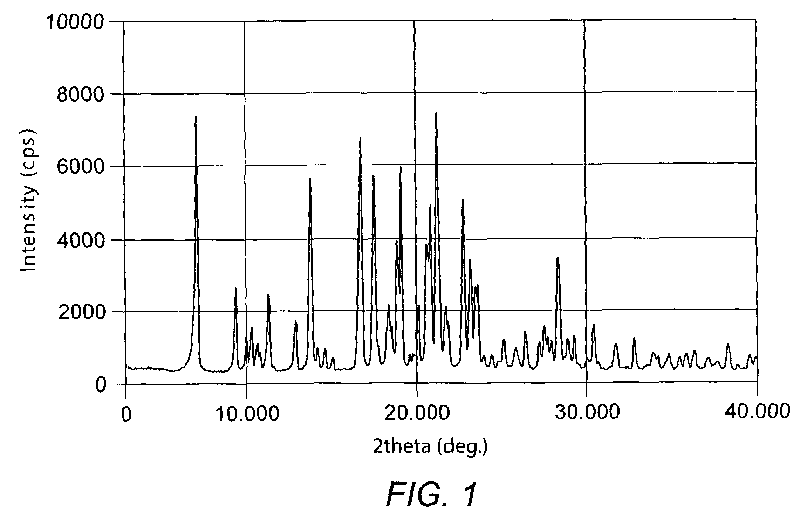

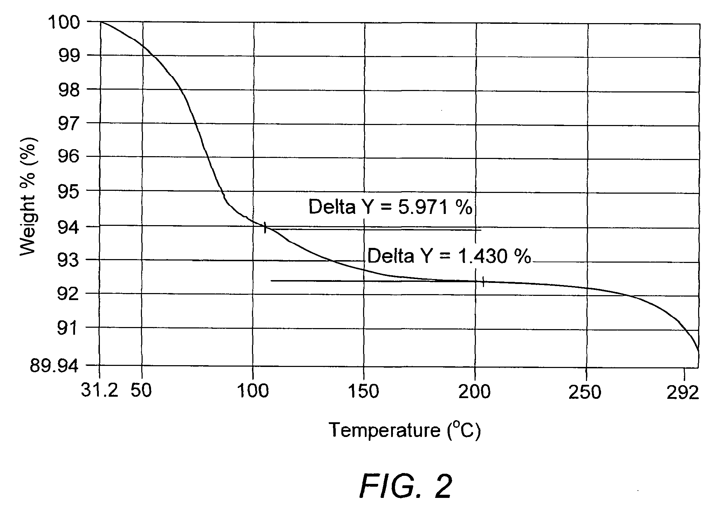

The present invention provides Form C characterised by powder X-ray diffraction spectrum as shown in Fig. 1. Form C is further characterized by thermogravimetric curve as shown in Fig. 2.

The present invention further provides a process for preparation of Form C having water content in range of 7 to 8.5%, as determined by the Karl Fischer method. Also the present invention provides a pharmaceutical composition comprising Form C of darunavir with one or more pharmaceutically acceptable excipients.

### Brief Description of the Drawings

Fig. 1 shows X-ray powder diffractogram (XRD) of darunavir Form C of the present invention.
Fig. 2 shows thermogravimetric curve (TG) of darunavir Form C of the present invention.
Fig. 3 shows X-ray powder diffractogram (XRD) of amorphous darunavir of the present invention.

### Description of the Invention

The invention will now be described in detail in connection with certain preferred and optional embodiments, so that various aspects thereof may be more fully understood and appreciated.

The present invention provides a crystalline form of darunavir (Form C) which is essentially a true hydrate as defined above. It is a stable polymorphic form that does not undergo any polymorphic transition under various humid conditions.

Crystalline darunavir hydrate Form C of the present invention comprises 1 to 3 molecules of water, which corresponds to a water content ranging from about 7.0 to 8.5% and further the crystalline darunavir hydrate of the present invention is substantially free of any organic solvent

Form C of the present invention is essentially free of any pseudopolymorphs or solvates disclosed in the prior art.

The term "substantially free" as used throughout this specification refers to darunavir Form C with residual solvent content as per ICH guidelines. However, the darunavir Form C of the present invention has residual solvent content not more than 500 ppm.

The crystalline nature of Form C of darunavir is analyzed, characterized and differentiated by X-ray diffractogram.

The X-ray powder diffraction pattern of the hydrated form of present invention was measured on a Rigaku Dmax 2200 advanced X-ray powder diffractometer with a copper-K-α radiation source. Darunavir hydrate Form C of the present invention has X-ray powder diffractogram pattern with peaks at 2θ values as listed in Table 1.

**Table 1: XRD values of Darunavir Form C**

| **Diffraction angles (± 0.2 2θ°)** | **Relative Intensity** |
|---|---|
| 7.12 | 100 |
| 9.42 | 34 |
| 10.04 | 13 |
| 10.34 | 16 |
| 11.36 | 30 |
| 12.94 | 20 |
| 13.86 | 76 |
| 16.78 | 90 |
| 17.54 | 75 |
| 18.38 | 23 |
| 18.56 | 14 |
| 18.90 | 50 |
| 19.14 | 79 |
| 20.14 | 23 |
| 20.64 | 46 |
| 20.88 | 60 |
| 21.28 | 97 |
| 21.74 | 21 |
| 21.92 | 14 |
| 22.84 | 65 |
| 23.20 | 41 |
| 23.50 | 29 |
| 23.68 | 30 |
| 25.20 | 12 |
| 26.44 | 15 |
| 27.54 | 15 |
| 28.34 | 42 |
| 29.28 | 12 |
| 30.44 | 18 |
| 32.80 | 12 |

The X-ray powder diffraction spectrum of darunavir Form C is depicted in Figure 1.

The present invention further provides a process for the preparation of Form C wherein 1-methylpyrrolidine-2,5-dione(3R,3aS,6aR)-tetrahydro-2H-furo[2,3-b]furan-3-yl carbonate is reacted with 4-amino-N-(3-amino-2-hydroxy-4-phenylbutyl)-N-isobutylbenzenesulfonamide to obtain a residue which is stirred in a mixture of water miscible solvent and water to obtain a solid which is dried to obtain darunavir hydrate Form C.

The present invention also provides a process of converting the pseudopolymorphs or solvates of darunavir to a stable polymorph Form C wherein the pseudopolymorph or solvate is stirred in a mixture of water miscible solvent and water and then filtered. The resulting solid is further stirred in water, filtered and dried to obtain Form C.

The water miscible solvent used in the process of present invention may be selected from methanol, ethanol, propanol, isopropanol, acetone, etc., but preferably the solvent used is methanol.

The solid product obtained on filtration is preferably dried under vacuum. The solid product is preferably dried at a temperature of not more than 40°C. The darunavir Form C of the present invention has a water content between 7.0 to 8.5%, as determined by the Karl Fischer method.

The residual solvent content of darunavir Form C was measured by gas chromatography using USP phase G27 column. From the result obtained it could be concluded that the crystalline darunavir hydrate Form C of the present invention is substantially free of any organic solvent.

The thermogravimetric analysis, indicating the change in mass as the sample is heated, cooled or held constant, of darunavir hydrate Form C of present invention showed about 5.5 - 7.5% weight loss. The thermogravimetric curve of darunavir Form C is depicted in Figure 2.

Further, the Form C of the present invention on drying at elevated temperature is converted to stable, non-hygroscopic amorphous darunavir which forms another aspect of the present invention.

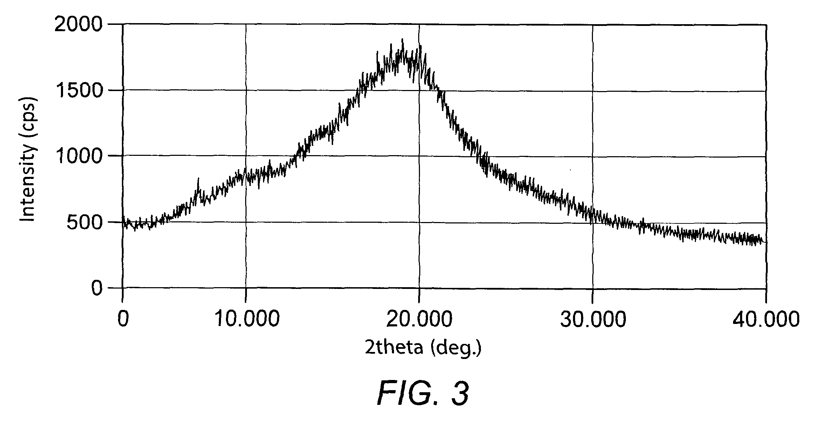

The amorphous darunavir obtained by the process of present invention is characterised by XRD pattern which is provided in Figure 3.

The present invention also relates to a pharmaceutical composition comprising crystalline darunavir Form C and a pharmaceutically acceptable carrier or diluent.

Also the present invention relates to a pharmaceutical composition comprising amorphous darunavir prepared by the process of present invention and a pharmaceutically acceptable carrier or diluent.

The present invention will now be further illustrated by reference to the following examples, which do not limit the scope of the invention any way.

### Examples

### Example 1 -

15 g of 1-methylpyrrolidine-2,5-dione(3R,3aS,6aR)-tetrahydro-2H-furo[2,3-b]furan-3-yl carbonate and 26 g of 4-amino-N-(3-amino-2-hydroxy-4-phenylbutyl)-N-isobutylbenzene sulfonamide were dissolved in dichloromethane (140 ml) at 25-30°C. The reaction mass was then cooled to -5 to 0°C. Triethylamine (16.7 ml) was added at 0°C under stirring. The temperature was then raised to 25°C and reaction mass was stirred for 8-10 hours. The reaction contents were quenched with saturated sodium bicarbonate (116 ml) and stirred. The organic layer was washed with water (50×2 ml) and concentrated under reduced pressure. To the above material isopropanol was added and contents heated to reflux. The reaction was cooled to 25-30°C and filtered. In the obtained solid a mixture of toluene and methanol (3:1) was added and reaction mass was heated to 65 ± 2°C. The reaction mass was chilled, filtered and then washed with toluene to obtain 33.6 g of darunavir.

### Example 2 -

Darunavir (5 g), obtained from Example 1, methanol (15 ml) and water (15 ml) was stirred for 1 hour in a reactor. The resulting slurry was filtered to obtain a wet cake which was suspended in 90 ml of water and stirred at 25-30°C for 8 - 10 hours. The contents were filtered and dried under vacuum at 35 - 40°C to obtain 4.0 g of darunavir Form C (K_{f} is 7.5%; yield - 78.12%), having an XRD pattern as shown in Fig 1.

### Example 3 -

5 g of darunavir, obtained in Example 1, was dissolved in 20 ml isopropanol and refluxed for 1 hour to obtain a clear solution. It was cooled to 10-15°C, filtered and dried under vacuum. The dried solid was dissolved in methanol (15 ml). To this solution 15 ml water was added and contents were stirred for 1 hour. The slurry was filtered. The wet cake was suspended in water (15 ml) and stirred at 25-35°C for 8-10 hours. The resulting solid was filtered and dried under vacuum at 35 - 40°C to obtain 4.2 g of hydrated darunavir Form C (K_{f} is 7.7%; yield - 82.03%).

### Example 4 -

7 g of darunavir isopropanolate (referred as Form J in US20050250845) was suspended in 21 ml methanol. Water (21 ml) was added and contents stirred for 1 hour. The resulting slurry was filtered. The wet cake thus obtained was suspended in water (20 ml) and stirred at 25-30°C for 10 hours. The contents were filtered and dried under vacuum at 35 - 40°C to obtain 4.8 g darunavir Form C (K_{f} is 7.8 %; yield - 77.54%).

Similarly, the other pseudopolymorphs reported in US'845 can be converted to darunavir Form C by following the above process.

### Example 5 -

Darunavir Form C obtained from any of the examples 2 to 4, when dried under vacuum at 60 ± 2°C for 8 hours yielded stable and non-hygroscopic amorphous darunavir, having an XRD pattern as shown in Fig 3.

### Example 6 - Hygroscopicity study

Darunavir Hydrate From C was subjected to hygroscopicity study and the test was performed as per the method given in British Pharmacopoeia.
57.6456 g of darunavir hydrate Form C was exposed at 25°C and 85% RH (relative humidity) for 24 hours and the weight of the sample was recorded as 57.6459 g. A weight change of 0.0003 g was observed which correspond to 0.06% weight change. As per the test a sample is of non-hygroscopic nature if the weight change observed is less than 0.2%. From the results it can be concluded that darunavir hydrate Form C is a non-hygroscopic material, suitable for pharmaceutical preparation.

### Example 7 - Stability study

Darunavir hydrate Form C was subjected to long term storage stability (6 months) under normal condition i.e 30 ± 2°C and 65 ± 5% RH (relative humidity) and under accelerated conditions i.e 40 ± 2°C and 75 ± 5 % RH (relative humidity) and it was found that there was no significant increase or decrease in the moisture content or HPLC purity of darunavir hydrate form C. Thus indicating that the Form C of the present invention is stable and suitable for pharmaceutical use.

## Claims

1. Crystalline darunavir hydrate comprising not more than 500 ppm of non-aqueous solvent, **characterised in that** the crystalline darunavir hydrate has a water content in the range of 7.0 to 8.5%, and is non-hygroscopic.

2. Crystalline darunavir hydrate according to claim 1, comprising not more than 500 ppm of methanol, methylene dichloride, ethyl acetate, tetrahydrofuran, triethylamine, toluene and ethanol.

3. Crystalline darunavir hydrate according to claim 1, having an XRD pattern in accordance with the following table, wherein the X-ray diffraction is measured with a copper-K-α radiation source:
| **Diffraction angles (± 0.2 2θ°)** | **Relative Intensity** |
|---|---|
| 7.12 | 100 |
| 9.42 | 34 |
| 10.04 | 13 |
| 10.34 | 16 |
| 11.36 | 30 |
| 12.94 | 20 |
| 13.86 | 76 |
| 16.78 | 90 |
| 17.54 | 75 |
| 18.38 | 23 |
| 18.56 | 14 |
| 18.90 | 50 |
| 19.14 | 79 |
| 20.14 | 23 |
| 20.64 | 46 |
| 20.88 | 60 |
| 21.28 | 97 |
| 21.74 | 21 |
| 21.92 | 14 |
| 22.84 | 65 |
| 23.20 | 41 |
| 23.50 | 29 |
| 23.68 | 30 |
| 25.20 | 12 |
| 26.44 | 15 |
| 27.54 | 15 |
| 28.34 | 42 |
| 29.28 | 12 |
| 30.44 | 18 |
| 32.80 | 12 |

4. A pharmaceutical composition comprising crystalline darunavir hydrate according to any one of claims 1 to 3, optionally in combination with one or more pharmaceutically acceptable excipients.

5. Crystalline darunavir hydrate according to any one of claims 1 to 3 for use in treating an HIV infection.

6. The use of crystalline darunavir hydrate according to any one of claims 1 to 3 in the manufacture of a medicament for use in treating an HIV infection.

7. A process for preparing darunavir hydrate comprising stirring darunavir, having at least one non-aqueous solvent in the crystal lattice, in a mixture of water miscible solvent and water; then filtering the mixture to obtain a solid; stirring the solid in water; filtering the solid/water mixture and drying to obtain crystalline darunavir hydrate according to any one of claims 1 to 3.

8. A process for preparing darunavir hydrate according to claim 7, wherein the water miscible solvent is methanol.

9. A process for preparing amorphous darunavir comprising drying crystalline darunavir hydrate prepared according to claim 7 or 8 at elevated temperature to obtain amorphous darunavir.

## Patentansprüche

1. Kristallines Darunavir-Hydrat, das maximal 500 ppm nichtwässriges Lösungsmittel beinhaltet, **dadurch gekennzeichnet, dass** das kristalline Darunavir-Hydrat einen Wassergehalt im Bereich von 7,0 bis 8,5 % hat und nicht hygroskopisch ist.

2. Kristallines Darunavir-Hydrat nach Anspruch 1, das maximal 500 ppm Methanol, Methylendichlorid, Ethylacetat, Tetrahydrofuran, Triethylamin, Toluol und Ethanol enthält.

3. Kristallines Darunavir-Hydrat nach Anspruch 1 mit einem XRD-Diagramm gemäß der folgenden Tabelle, wobei die Röntgenbeugung mit einer Kupfer-K-α-Strahlungsquelle gemessen wird:
| **Beugungswinkel (± 0,2 2θ°)** | **Relative Intensität** |
|---|---|
| 7,12 | 100 |
| 9,42 | 34 |
| 10,04 | 13 |
| 10,34 | 16 |
| 11,36 | 30 |
| 12,94 | 20 |
| 13,86 | 76 |
| 16,78 | 90 |
| 17,54 | 75 |
| 18,38 | 23 |
| 18,56 | 14 |
| 18,90 | 50 |
| 19,14 | 79 |
| 20,14 | 23 |
| 20,64 | 46 |
| 20,88 | 60 |
| 21,28 | 97 |
| 21,74 | 21 |
| 21,92 | 14 |
| 22,84 | 65 |
| 23,20 | 41 |
| 23,50 | 29 |
| 23,68 | 30 |
| 25,20 | 12 |
| 26,44 | 15 |
| 27,54 | 15 |
| 28,34 | 42 |
| 29,28 | 12 |
| 30,44 | 18 |
| 32,80 | 12 |

4. Pharmazeutische Zusammensetzung, die kristallines Darunavir-Hydrat nach einem der Ansprüche 1 bis 3 umfasst, optional in Kombination mit einem oder mehreren pharmazeutisch akzeptablen Hilfsstoffen.

5. Kristallines Darunavir-Hydrat nach einem der Ansprüche 1 bis 3 zur Verwendung bei der Behandlung einer HIV-Infektion.

6. Verwendung von kristallinem Darunavir-Hydrat nach einem der Ansprüche 1 bis 3 bei der Herstellung eines Medikaments zur Verwendung bei der Behandlung einer HIV-Infektion.

7. Verfahren zur Herstellung von Darunavir-Hydrat, das das Rühren von Darunavir, mit wenigstens einem nichtwässrigen Lösungsmittel im Kristallgitter, in einem Gemisch von in Wasser mischbarem Lösungsmittel und Wasser; dann Filtern des Gemischs zum Erhalten eines Feststoffs; Rühren des Feststoffs in Wasser; Filtern des Feststoff/Wasser-Gemischs und Trocknen zum Erhalten von kristallinem Darunavir-Hydrat nach einem der Ansprüche 1 bis 3 beinhaltet.

8. Verfahren zur Herstellung von Darunavir-Hydrat nach Anspruch 7, wobei das in Wasser mischbare Lösungsmittel Methanol ist.

9. Verfahren zur Herstellung von amorphem Darunavir, das das Trocknen von kristallinem Darunavir-Hydrat, hergestellt nach Anspruch 7 oder 8, bei erhöhter Temperatur beinhaltet, um amorphes Darunavir zu erhalten.

## Revendications

1. Darunavir cristallin hydraté comprenant pas plus de 500 ppm d'un solvant non aqueux, **caractérisé en ce que** le darunavir cristallin hydraté a une teneur en eau dans la gamme de 7,0 à 8,5 % et est non hygroscopique.

2. Darunavir cristallin hydraté selon la revendication 1, comprenant pas plus de 500 ppm de méthanol, de dichlorure de méthylène, d'acétate d'éthyle, de tétrahydrofurane, de triéthylamine, de toluène et d'éthanol.

3. Darunavir cristallin hydraté selon la revendication 1, ayant un diagramme de DRX selon le tableau suivant, dans lequel la diffraction des rayons X est mesurée avec une source utilisant le rayonnement Kα du cuivre :
| **Angles de diffraction (± 0,2 2θ°)** | **Intensité relative** |
|---|---|
| 7,12 | 100 |
| 9,42 | 34 |
| 10,04 | 13 |
| 10,34 | 16 |
| 11,36 | 30 |
| 12,94 | 20 |
| 13,86 | 76 |
| 16,78 | 90 |
| 17,54 | 75 |
| 18,38 | 23 |
| 18,56 | 14 |
| 18,90 | 50 |
| 19,14 | 79 |
| 20,14 | 23 |
| 20,64 | 46 |
| 20,88 | 60 |
| 21,28 | 97 |
| 21,74 | 21 |
| 21,92 | 14 |
| 22,84 | 65 |
| 23,20 | 41 |
| 23,50 | 29 |
| 23,68 | 30 |
| 25,20 | 12 |
| 26,44 | 15 |
| 27,54 | 15 |
| 28,34 | 42 |
| 29,28 | 12 |
| 30,44 | 18 |
| 32,80 | 12 |

4. Composition pharmaceutique comprenant du darunavir cristallin hydraté selon l'une quelconque des revendications 1 à 3, éventuellement en combinaison avec un ou plusieurs excipients pharmaceutiquement acceptables.

5. Darunavir cristallin hydraté selon l'une quelconque des revendications 1 à 3, pour une utilisation dans le traitement d'une infection par le VIH.

6. Utilisation de darunavir cristallin hydraté selon l'une quelconque des revendications 1 à 3 dans la fabrication d'un médicament pour une utilisation dans le traitement d'une infection par le VIH.

7. Procédé de préparation de darunavir hydraté comprenant l'agitation de darunavir, ayant au moins un solvant non aqueux dans sa structure cristalline, dans un mélange d'un solvant miscible à l'eau et d'eau; puis la filtration du mélange pour obtenir un solide; l'agitation du solide dans de l'eau; la filtration du mélange solide/eau et son séchage pour obtenir du darunavir cristallin hydraté selon l'une quelconque des revendications 1 à 3.

8. Procédé de préparation de darunavir hydraté selon la revendication 7, dans lequel le solvant miscible à l'eau est le méthanol.

9. Procédé de préparation de darunavir amorphe comprenant le séchage de darunavir cristallin hydraté préparé selon la revendication 7 ou 8, à haute température, pour obtenir du darunavir amorphe.
